# EUROPEAN PATENT APPLICATION

(11) **EP 2 394 976 A1**
(43) Date of publication of application: **14.12.2011**
(21) Application number: 10165742.7
(22) Date of filing: 11.06.2010
(51) Int. Cl.: C07B 41/02, C07C 213/08, C07C 215/64, C07C 45/67, C07C 49/84, C07C 201/12, C07C 205/25, C07C 253/30, C07C 255/53

(54) **Process for demethylating aromatic methyl ethers using 3-mercaptopropionic acid**

(71) Applicant: LEK Pharmaceuticals d.d., 1526 Ljubljana (SI)
(72) Inventor: The designation of the inventor has not yet been filed
(74) Representative: Kunic Tesovic, Barbara

(57) **Abstract**

The present application discloses a process for demethylating aromatic methyl ethers by reaction with 3-mercaptopropionic acid or salts thereof. One preferred example is the demethylation of venlafaxine forming O-desmethylvenlafaxine.

## Description

### Field of Invention

The present invention relates to the field of preparative organic chemistry, in particular to a new process of demethylating aromatic methyl ethers, such as for example a process of demethylating venlafaxine, a precursor for *O*-desmethylvenlafaxine.

### Background of the Invention

The cleavage of aromatic alkyl ethers is a versatile transformation in organic synthesis as it furnishes the widespread compound class of phenols and is therefore one useful member of the huge family of protecting and deprotecting reactions. Phenols are of special interest since these compounds play an important role in the manufacture of natural products, pharmaceuticals, drugs and fine chemicals. Due to the high reactivity of a phenyl hydroxyl group it is often necessary to temporarily protect it during multi-step syntheses. Among phenol protecting groups, the simple methyl group combines many advantages including stability and compatibility with various reaction conditions. It is therefore important to have suitable and efficient methods for the deprotection of aromatic methyl ethers. One way to effectively demethylate aromatic methyl ether is related to the use of sulfur or halide based compounds.

Halide containing demethylating methods for aromatic methyl ethers were reported using iodoalkyls (Tetrahedron Letters 2008, 4054) or a mixture of silicon tetrachloride, lithium iodide and boron trifluoride (Tetrahedron Letters 2004, 3729). Sulfur containing demethylating agents have been reported manifold and include methyl sulfurous acid (J. Med. Chem. 2004, 2365; J. Org. Chem. 1987, 5143) in the presence of methionine. Lithium ethylthiolate was tested as a demethylating agent for aromatic methyl ethers with and without the assistance of microwave heating (Synlett 2008, 1993). Thiophenol and 2-amino-thiophenol were used in demethylating reactions with several methoxy naphthalines and diversely substituted phenyl methyl ethers as substrates (J. Org. Chem., 2002, 6406).

On the limited substrate scope of venlaflaxine the applicability of dodecanethiol and its sodium salt, as well as benzenethiol sodium salt (WO 03/048104 A1); trimethylsilyl chloride ion the presence of sodium sulfide (WO 2009/034434 A2); 1,2-ethane dithiol, 2-diethylaminoethane thiol and sodium thiol (WO 2009/053731 A1); sodium sulfide anhydrous and as hydrate as well as in the presence and in the absence of selenium (WO 2007/071404 A1) and 2-(diethylamino)ethanothiol (WO 2008/090465 A2) was tested. On a wider scope of substituted methoxy naphthalines and substituted phenyl methyl ethers 2-(diethylamino)ethanethiol hydrochloride was used as a demethylating agent (J. Org. Chem. 2006, 7103). For demethylating venlafaxine it has also been reported the use of thioglycolic acid, thioacetic acid and of thioglycoamide and salts thereof (WO 2009/084038 A2).

Due to the wide range of phenols and the peculiarity of organic synthesis to provide efficient reaction systems while paying attention to a high variety of reaction substrates there is a need for new and practical methodologies to demethylate aromatic methyl ethers.

It is therefore an object of the present invention to provide a new demethylation agent for aromatic methyl ethers, which is capable of demethylating a broad range of aromatic methyl ethers including venlafaxine as a pharmaceutically relevant substrate.

### Summary of the Invention

Aspects, advantageous features and preferred embodiments of the present invention summarized in the following items, respectively alone or in combination, contribute to solving this and other objects of the invention:
1. A process for demethylating aromatic methyl ethers of the formula (I), 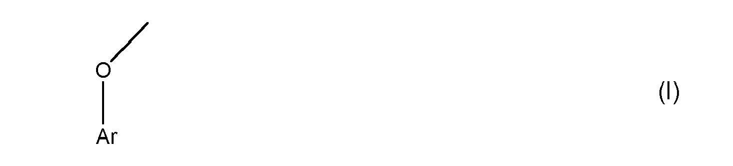 wherein Ar is selected from the group consisting of substituted or unsubstituted aryl and substituted or unsubstituted heteroaryl groups;
   the process comprising reacting an aromatic methyl ether of the formula (I) with 3-mercaptopropionic acid [formula (II)] or salts thereof 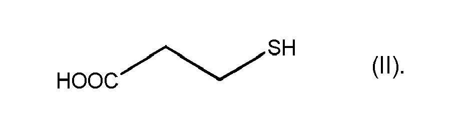
2. The process according to item 1, wherein the reaction is carried out in the presence of a base in an organic solvent or mixtures comprising organic solvents.
3. The process according to item 1 and 2, further comprising isolating the demethylated product from the reaction mixture.
4. The process according to item 3, wherein the isolation step comprises a method selected from the group of filtration, extraction, precipitation and crystallization.
5. The process of item 3, wherein the isolation step comprises a method of extraction and column chromatography.
6. The process according to any one of the preceding items, wherein the demethylated product is separated from unreacted aromatic methyl ether of the formula (I) by precipitation of unreacted aromatic methyl ether of the formula (I) in a basic mixture.
7. The process according to any one of the preceding items, wherein the demethylated product is obtained in greater than 99 % HPLC chromatographic purity.
8. The process according to any one of the preceding items, wherein the aromatic methyl ether (I) has n (n>1) methoxy groups as substituents on the aryl or heteroaryl groups, preferably 2, but only one methoxy group is selectively demethylated.
9. The process according to any one of items 1-7, wherein the aromatic methyl ether (I) has n (n>1) methoxy groups as substituents on the aryl or heteroaryl groups, preferably 2, and 2 and optionally up to n methoxy groups are demethylated.
10. The process according to any one of the preceding items, wherein Ar consists of optionally substituted aryl groups comprising phenyl and naphthyl.
11. The process according to any one of the preceding items, wherein Ar is substituted.
12. The process of item 11, wherein Ar is substituted and at least one substituent is selected from the group of electron donating substituents consisting of respectively substituted or unsubstituted alkyls, amines, amides, hydroxyls and ethers, preferably alkyls bearing an amino group in α-position.
13. The process of item 11, wherein Ar is substituted and at least one substituent is selected from the group of electron withdrawing substituents consisting of halogens, nitro groups, nitriles, carbonyls and carboxyls, preferably nitriles, carbonyls and nitro groups.
14. The process of item 11, wherein Ar is substituted with n (n>1) substituents and at least one substituent is selected from the group of electron donating substituents as described under item 12, and at least one another substituent is selected from the group of electron withdrawing substituents as under item 13.
15. The process according to any one of the preceding items, wherein Ar is substituted and the substituent is selected from the group of 5-cyano-3-methoxyphenyl, 4-cyano-2,6-methoxyphenyl, 4-(4-methoxybenzoyl)phenyl or 4-cyanonaphthyl and 4-nitronaphthyl.
16. The process according to any one of items 1 to 15, wherein Ar is substituted by a residue containing an amino group and the amino group is available in salt form, preferably as hydrochloride salt.
17. The process according to item 1, wherein Ar is substituted by a *p*-(dimethylamino)(1-hydroxycyclohexyl)methyl group.
18. The process according to any of the preceding items, wherein the reaction is carried out in a solvent selected from the group consisting of dimethylformamide, *N-*methyl-2-pyrrolidone, ethers, polyethers, polyethylene glycols, hydrocarbons, and *N,N'-*dimethylacetamide, preferably *N,N'*-dimethylacetamide.
19. The process according to any of the preceding items, wherein the reaction temperature of the demethylation is in a range from 20 °C to 250 °C, preferably from 50 °C to 230 °C, more preferably from 150 °C to 200 °C.
20. The process according to any of the preceding items, wherein a base is used for the demethylation.
21. The process according to item 20, wherein the base used for the demethylation is selected from the group consisting of inorganic basic salts comprising carbonates, hydrides and hydroxides.
22. The process according to item 21, wherein the base used for the demethylation is selected from the group consisting of carbonates comprising alkaline, alkaline earth and earth metal carbonates, preferably potassium carbonate and sodium carbonate, most preferably potassium carbonate.
23. The process according to item 21, wherein the base used for the demethylation is selected from the group consisting of hydrides comprising alkali hydrides, preferably sodium hydride.
24. The process according to item 21, wherein the base used for the demethylation is selected from the group consisting of hydroxides comprising alkali hydroxides, preferably sodium hydroxide.
25. The process according to item 20, wherein a base used for the demethylation is selected from the group consisting of organic bases such as amides, amidines, tertiary amines including pyridine, triethylamine.
26. The process according to any of the preceding items, wherein the demethylation is carried out in a pressurizable reaction vessel, preferably an autoclave.
27. The process according to item 26, wherein an autoclave is used to run the demethylation at a temperature beyond the boiling point of the corresponding solvent at atmospheric pressure, preferably beyond the boiling point of *N,N'*-dimethylacetamide at atmospheric pressure.
28. The process according to any of the preceding items further comprising subjecting the demethylated product of the aromatic methyl ether (I) to further reaction steps to obtain a pharmaceutical active compound.
29. The use of 3-mercaptopropionic acid [formula (II)] or salts thereof for demethylating of venlafaxine or a salt thereof.
30. A process for the preparation of a pharmaceutical composition containing the demethylated product of the aromatic methyl ether (I) or a salt thereof as an active ingredient comprising :
   (i) converting an aromatic methyl ether (I) or a salt thereof to the demethylated product or salt thereof by a process according to any one of item 1 to 27;
   (ii) optionally further reaction according to item 28;
   (iii) optionally forming a finally suitable salt;
   (iv) formulating the obtained demethylated product or salt thereof with at least one pharmaceutical acceptable excipient.
31. A process for the preparation of a pharmaceutical composition containing *O*-desmethylvenlafaxine or a salt thereof as an active ingredient comprising:
   (i) converting venlaflaxine or a salt thereof to *O*-desmethylvenlaflaxine or salt thereof by a process according to any one of claim 1 to 27 or use according to 29;(ii) optionally forming a finally suitable salt;
   (iii) formulating the obtained *O*-desmethylvenlaflaxine or salt thereof with at least one pharmaceutical acceptable excipient.

### Detailed Description of the Invention

The present invention is now described in more detail by preferred embodiments and examples, which are however presented for illustrative purpose only and shall not be understood as limiting the scope of the present invention in any way.

The present invention provides an industrially applicable, economical and acceptable synthetic process introducing 3-mercaptopropionic acid as demethylating agent for aromatic methyl ethers (Figure 1). 3-mertcaptopropionic acid is a new reagent for efficient demethylation of aromatic methyl ethers and is applicable to the synthesis of *O*-desmethylvenlafaxine, the major metabolite of venlafaxine, an active pharmaceutical ingredient indicated for the treatment of major depressive disorder. It could have been shown that *O*-desmethylvenlafaxine inhibits the norepinephrine and serotonin uptake and can therefore be considered as an active pharmaceutical ingredient itself.

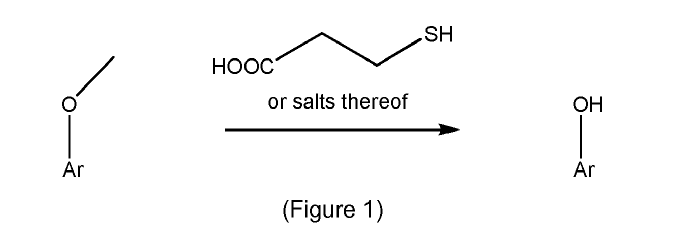

The process as disclosed herein is not limited to venlafaxine as the only substrate. Differently substituted aromatic methyl ethers were applied to the similar reaction conditions and successful conversion to the corresponding demethylated product was observed as described below. Hence the process according to the present invention allows a wide and useful applicability of demethylation of aromatic methyl ethers. Further advantages that are made possible by the process according to the present invention are described below.

The term "alkyl" as used herein, if not stated otherwise with respect to particular embodiments, includes reference to a straight or branched chain alkyl moiety having 1, 2, 3, 4, 5 or 6 carbon atoms. This term includes methyl, ethyl, propyl (*n-*propyl or isopropyl), butyl (*n*-butyl, *sec*-butyl or *tert*-butyl), pentyl, hexyl and the like. In particular, alkyl may have 1, 2, 3 or 4 carbon atoms.

The term "aryl" as used herein, if not stated otherwise with respect to particular embodiments, includes reference to an aromatic ring system comprising 6, 7, 8, 9, 10, 11, 12, 13, 14, 15 or 16 ring carbon atoms. Aryl is often phenyl but may be a polycyclic ring system, having two or more rings, at least one of which is aromatic. This term includes phenyl, naphthyl, fluorenyl, azulenyl, indenyl, anthryl and the like.

The term "heteroaryl" as used herein includes, if not stated otherwise with respect to particular embodiments, an unsaturated, aromatic heterocyclic ring moiety having 3, 4, 5, 6, 7, 8, 9, 10, 11, 12, 13, 14, 15 or 16 ring atoms, at least one of which is selected from nitrogen, sulfur and oxygen. Furthermore, the term "heteroaryl" may include an aromatic heterocyclic ring system having 5, 6, 7, 8, 9, 10, 11, 12, 13, 14, 15 or 16 ring atoms, at least one of which is selected from nitrogen, sulfur and oxygen. The group may be a polycyclic ring system, having two or more rings, at least one of which is aromatic, but is more often monocyclic. This term includes pyrimidinyl, furanyl, benzo[b]thiophenyl, thiophenyl, pyrrolyl, imidazolyl, pyrrolidinyl, pyridinyl, benzo[b]furanyl, pyrazinyl, purinyl, indolyl, benzimidazolyl, quinolinyl, phenothiazinyl, triazinyl, phthalazinyl, oxazolyl, isoxazolyl, thiazolyl, isoindolyl, indazolyl, purinyl, isoquinolinyl, quinazolinyl, pteridinyl and the like.

The term "substituted" as used herein in reference to a structure/moiety/group means that one or more, especially up to 5, more especially 1, 2 or 3, of the hydrogen atoms in said structure/moiety/group are replaced independently of each other by the corresponding number of substituents known to a person skilled in the art. Typical substituents include, without being limited to halogen, cyano, nitro, oxo, -NR', -OR', -C(O)R', -C(O)OR', -OC(O)R', -S(O)R', - N(R')R", -CHNR'R", -C(O)N(R')R", -SO₂N(R')R' and R"', wherein each of R', R" and R'" are selected from the group consisting of, optionally cyclic, C1 - C6 alkyl, C1 - C6 alkoxy, aryl, amino and each R' and R" may be, optionally and independently further substituted with one or more of, hydrogen, halogen, cyano, amino, hydroxyl, C1 - C6 alkyl and C1 - C6 alkoxy. Specific substituents in particular include nitro, cyano, amino, hydroxyl, optionally cyclic C1 - C6 alkyl and C1 - C6 alkoxy. It will be understood that substituents are at positions where they are chemically possible, it being known or evident to the person skilled in the art to decide (either experimentally or theoretically) without inappropriate effort whether a particular substitution is possible. For example, substituents which may be unstable or may affect reactions disclosed herein may be omitted, at least at the relevant stage of intermediate compound or of the affected reaction.

The term "base" used herein according to preferred embodiments can be any base known and typically used in organic synthesis. The base can include, without being limited to amides, amidines, tertiary amines including pyridine, triethylamine and the like. The preferred base is selected from inorganic basic salts, such as carbonates comprising alkaline, alkaline earth and earth metal carbonates including potassium carbonate, sodium carbonate; hydrides such as sodium hydride, hydroxides such as sodium hydroxide, phosphates, preferably sodium hydroxide as a 2M aqueous solution, most preferably potassium carbonate.

According to the present invention a process is provided for demethylating aromatic methyl ethers of the formula (I), 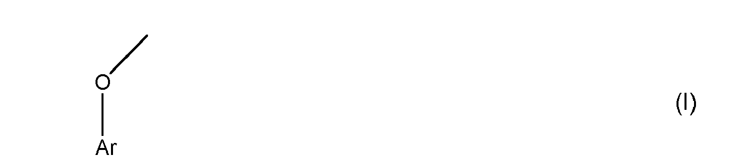 wherein Ar is selected from the group consisting of substituted or unsubstituted aryl and substituted or unsubstituted heteroaryl groups;
by reacting an aromatic methyl ether of the formula (I) with 3-mercaptopropionic acid [formula (II)] or salts thereof. By using 3-mercaptopropionic acid this process utilizes a cheap chemical that is readily available at prevalent vendors for a phenol or analogous deprotection reaction, an often used synthetic tool in organic and inorganic syntheses. The process of the present invention is therefore useful as an efficient procedure for the synthesis of aromatic phenols. Furthermore, by using 3-mercaptopropionic acid, it turned out that the demethylation agent and the corresponding methyl ethers can easily be removed from the reaction mixture, if desired, by a single extraction step with basic aqueous solution due to both the carboxylic and the thiol functional group.

Surprisingly, when applying the procedural concept in a preferred embodiment to demethylate venlafaxine, good yields combined with exceptional purity of the product compounds can be achieved. As a further advantage, the process according to the present invention is applicable on a broad range of aromatic methyl ethers covering a manifold compilation of compounds with diverse substitution pattern.

Moreover, 3-mercaptopropionic acid is a preferable demethylation agent, in that it is a weakly odorous thiol equivalent which makes working with it free of special precautions and much more convenient compared to commonly used and strongly odorous alkyl and aryl thiols.

The process according to the present invention is preferably carried out in the presence of a base in an organic solvent or mixtures thereof. Further, the reaction can be suitably carried out in polar aprotic organic solvents without however being limited thereto. In a preferred embodiment of the present invention the solvent for the demethylation reaction is further selected from the group consisting of dimethylformamide, *N-*methyl-2-pyrrolidone, ethers, polyethers, polyethylene glycols, hydrocarbons, and *N,N'*-dimethylacetamide, most preferably *N,N'*-dimethylacetamide.

The base preferably used in the reaction mixture can be chosen, but is not limited to, from prevalent inorganic bases as described above. The presence of a suitable base in the reaction renders the advantage of converting the demethylation reagent 3-mercaptopropionic acid into a salt by deprotonation, affording an odorless derivative.

In another preferred embodiment of the present invention 3-mercaptopropionic acid or salts thereof is used for demethylating venlafaxine or a salt thereof.

The demethylation concept of the present invention allows the reaction temperature to be set over a wide range, for example in a range from 20 °C to 250 °C, preferably from 50 °C to 230 °C, more preferably from 150 °C to 200 °C.

The demethylation is carried out in a suitable reaction vessel, preferably a pressurizable reaction vessel, more preferably an autoclave. The use of an autoclave facilitates running the demethylation at a temperature beyond the boiling point of the corresponding solvent at atmospheric pressure, preferably beyond the boiling point of *N,N'*-dimethylacetamide at atmospheric pressure.

One important aspect of the process according to the present invention suitably comprises the isolation of the desired demethylated product from the reaction mixture. Isolating the desired product comprises, but is not limited to, a method of filtration, extraction, precipitation and/or crystallization, optionally followed by another filtration. As a further significant advantage of the present invention, 3-mercaptopropionic acid and the corresponding mercapto methyl ether as the side product of the demethylation are water soluble in basic media, which offers the opportunity of separating the aforementioned compounds easily from nonpolar chemicals by one simple purification step. Therefore, filtration of the crude demethylation reaction mixture after the addition of water allows separating unreacted aromatic methyl ether and desired demethylated product from the residual mixture containing excess 3-mercaptopropionic acid and the corresponding mercapto methyl ether. Due to excess base substance such as K₂CO₃ the water phase is slightly basic and thus unreacted 3-mercaptopropionic acid and the corresponding mercapto methyl ether remain deprotonated and water soluble. In contrast to 3-mercaptopropionic acid and the corresponding mercapto methyl ether, the substrate compound of formula (I) and the desired demethylated product, such as venlafaxine and *O*-desmethylated venlafaxine, typically do not dissolve at slightly basic pH values and thus can easily be separated by filtration. In a preferred embodiment precipitated substrate and desired product such as venlafaxine and *O*-desmethylated venlafaxine are separated from the resulting aqueous reaction mixture.

Unreacted aromatic methyl ethers and desired demethylated products can be further separated from each other by adding the crude solid mixture to an aqueous base solution. The base selected for this purification step is supposed to be capable of deprotonating the hydroxyl group of a phenol in order to convert it into the corresponding phenolate. The same applies to other demethylated aryl-OH-compounds. In a preferred embodiment of the present invention this base is sodium hydroxide provided in a 2 molar aqueous solution.

Methylated aromatic ethers that did not dissolve can be removed from the residual product solution by filtration.

In a preferred embodiment unreacted aromatic methyl ethers such as venlafaxine is separated from the basic solution by filtration.

Subsequent extractions in order to wash the filtrate are conducted using a water/organic solvent phase system. The organic solvent for the aforementioned extraction is selected from a group of ethers, hydrocarbons, chlorinated hydrocarbons, esters and mixtures thereof. In a preferred embodiment ethers, ester or mixtures thereof are used, in a more preferred embodiment methyl *tert*-butyl ether is used, in another preferred embodiment a mixture of methyl *tert*-butyl ether and ethyl acetate is used. Extraction steps can be repeated if desired until a further extraction step is considered of having no meaningful additional impact on the purity of the water phase.

The process of the present invention allows precipitation of the demethylated desired product by adjusting the pH value of the solution to a level, whereat precipitation starts. In a preferred embodiment of the invention the pH value is adjusted to a range between 5 and 12. In a more preferred embodiment the pH value is in range between 5 and 9, in a most preferred embodiment the pH value is 7.5.

Acids for decreasing the pH value of the solution are selected from a group of prevalent inorganic acids comprising hydro halogenic acid including hydrochloric acid, sulfuric acid, phosphoric acid, nitric acid, acetic acid and glacial acetic acid, preferably acetic acid.

Additional crops of the demethylated product can be collected, if desired, by additional changing the pH value of the solution. In a preferred embodiment of the present invention the pH value of the solution is increased to 9.0 by addition of 1 molar aqueous solution of sodium hydroxide. In another preferred embodiment of the present invention the number of crops is at least one, in a more preferred embodiment the number of crops is two.

Purity of the product can be enhanced by subjecting the crude, precipitated product to crystallization. The crystallization method can be selected, but is not limited to, from a group consisting of diffusion method, evaporation method and thermic method, i.e. cooling of a saturated or thick solution of the desired product that is meant being crystallized. In a preferred embodiment of the present invention the desired product was crystallized by thermic crystallization.

In a preferred embodiment of the present invention the crystallization is carried out in a mixture of ethyl acetate:ethanol 1 : 4 with satisfactory yields in high purity. The purity determined by HPLC chromatography is preferably in a range between 95 to 100 %, more preferably between 99 to 100 %, most preferably between 99.9 to 100 %.

Remarkably, a purification method comprising only extraction (optionally washing), precipitation and crystallization, i.e. without chromatography, allows the efficient isolation of aromatic phenols such as e.g. *O*-desmethylvenlafaxine in very high purity.

If desired, isolating the demethylated product can also be accomplished by other methods comprising e.g. extraction as described above and column chromatography or combinations thereof. In such a case the use of 3-mercaptopropionic acid as demethylation reagent has the advantage that in the presence of a base deprotonation takes place and easy isolation using extraction or chromatography is feasible. After the demethylation reaction 3-mercaptopropionic acid and its methylated analogue (side product of demethylation) become ionic salts upon basic treatment e.g. with preferably aqueous K₂CO₃ while the demethylated desired product remains less polar. Ionic species such as salts do not elute easily with unpolar solvents during chromatographic separation while neutral or less polar compounds elute efficiently. The difference in polarity thus allows easy separation of the product and unreacted substrate from 3-mercaptopropionic acid and its methylated analogue both by extraction or chromatography.

Within the process according to the present invention the aromatic methyl ether (I) may have n (n>1) methoxy group as substituents on the aryl or heteroaryl groups, for example 2, but only one methoxy group may selectively be demethylated. The demethylation is thus effective on only one of all methoxy groups. Conversely, in case only one methoxy group is selectively demethylated, at least 50 %, preferably more than 75 %, more preferably more than 90 % of n methoxy groups remain methylated.

In an alternatively applicable embodiment of the present invention, in case the aromatic methyl ether (I) has n (n>1) methoxy groups as substituents on the aryl or heteroaryl groups, for example 2, and 2 and optionally up to n methoxy groups are demethylated. The demethylation is thus substantially effective on all n methoxy groups.

For example, in case n>1 and methoxy groups are attached to the aromatic ring Ar in equivalent positions, the amount of 3-mercaptopropionic acid can control selectivity for a mono- or polydemethylation. Demethylating one methoxy group increases the electron density within the ring and subsequent demethylation is disfavored. The process of the present invention however is capable of further demethylating, if desired, despite such an electronic deactivation. In a preferred embodiment of the present invention Ar is a phenyl ring and substituted with a cyano group and two competing methoxy groups in the meta positions and both the monodemethylation and the bidemethylation can be achieved by using an appropriate amount of 3-mercaptopropionic acid.

Furthermore, the process of the present invention allows influencing the selectivity of the demethylation by choosing electron donating or electron withdrawing substituents in a suitable position, for example ortho, meta or para within a phenyl ring, to electronically facilitate or to stall demethylation of a certain methoxy group. In a preferred embodiment of the present invention Ar is a phenyl ring and substituted with an electron withdrawing substituent and competing methoxy groups in any of such ortho, meta and para positions to the electron withdrawing group and if optionally methoxy groups are competing in meta and para or ortho position, the methoxy group in meta-position is disfavored for demethylation, wherein at least 40% yield and 60% conversion of para- or ortho-demethylated product is observed. In another preferred embodiment of the present invention Ar is a phenyl ring and substituted with an electron withdrawing substituent and three competing methoxy groups in both meta positions and para position to the electron withdrawing group and the methoxy group in para-position is selectively demethylated. In more preferred embodiment of the present invention Ar is a phenyl ring and substituted with cyano group and three competing methoxy groups in both meta positions and in para position and the methoxy group in para position is demethylated selectively.

The aromatic methyl ethers of the formula (I) according to the present invention have the aromatic group Ar which is optionally substituted, comprising phenyl, naphthyl, fluorenyl, azulenyl, indenyl, anthryl and the like or salts of all mentioned compound classes and substituted forms thereof. The optional substituent can be selected from a group of electron donating substituents consisting of respectively substituted or unsubstituted alkyls, amines, amides and/or selected from a group of electron withdrawing substituents consisting of respectively substituted or unsubstituted nitroxides, nitriles, carbonyls and carboxyls. In a preferred embodiment of the present invention one or more substituents are selected from the group of electron withdrawing substituents. In case Ar is substituted with one or more electron withdrawing substituents, high to moderate yields together with extraordinary conversions are observed upon applying the process according to the present invention to such substrates. In another preferred embodiment Ar is substituted, wherein the substituent is selected from the group of 5-cyano-3-methoxyphenyl, 4-cyano-2,6-methoxyphenyl, 4-(4-methoxybenzoyl)phenyl or 4-cyanonaphthyl and 4-nitronaphthyl.

Yet in a particular preferred embodiment of the present invention Ar is substituted by a residue containing an amine group and the amine group is available in salt form, preferably as hydrochloride salt, most preferably the substituent is *p-*(dimethylamino)(1-hydroxycyclohexyl)methyl as described in formula (III) and also denoted as venlafaxine

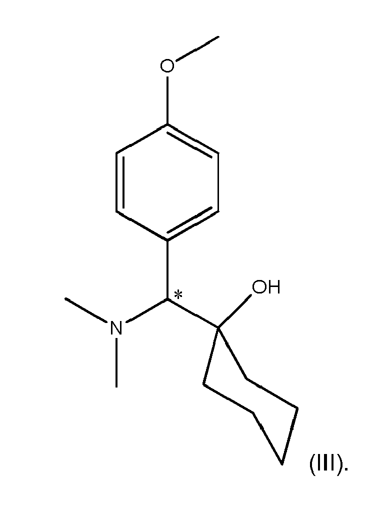

The compound of formula (III) comprises both enantiomers, wherein the stereogenic center marked with an * is either in (*R*)- or (*S*)-configuration at marked center, or it is in racemic form. Further, the compound of the formula (III) is not limited to a specific conformation of the cyclohexyl ring.

A novel and efficient synthetic route for *O*-desmethylvenlaflaxin is established which is obtained in convenient and industrial applicable manner upon using weakly odorous demethylation reagent by applying the process of the present invention on a *p-*(dimethyiamino)(1-hydroxycyclohexyl)methyl substituted phenyl group as in formula (III).

Another aspect of the present invention relates to subjecting the demethylated product of the aromatic methyl ether (I) to further reaction steps to obtain a desired pharmaceutical active compound, which may be different in structure from the primary demethylated product such as *O*-desmethylvenlafaxine.

Still another aspect relates to a process for the preparation of a pharmaceutical composition containing the demethylated product of the aromatic methyl ether (I) or a salt thereof as an active ingredient comprising:
(i) converting an aromatic methyl ether (I) or a salt thereof to the demethylated product or salt thereof by a process according to the present invention;
(ii) optionally forming a finally suitable salt
(iii) formulating the obtained demethylated product or salt thereof with at least one pharmaceutical acceptable excipient.

Pharmaceutically acceptable excipients may be selected from the group consisting of binders, diluents, disintegrating agents, stabilizing agents, preservatives, lubricants, fragrances, flavoring agents, sweeteners and other excipients known in the field of the pharmaceutical technology. Preferably, carriers and excipients may be selected from the group consisting of lactose, microcrystalline cellulose, cellulose derivatives, (e.g. hydroxypropylcellulose, croscarmellose sodium), polyacrylates, calcium carbonate, starch, colloidal silicone dioxide, anhydrous dibasic calcium phosphate, sodium starch glycolate, talc, magnesium stearate, sodium stearyl fumarate, mannitol, polyvinylpyrrolidone, polyethylene glycol and other excipients known in the field of the pharmaceutical technology

The pharmaceutical compositions according to the present invention may be prepared by methods known in the field of the pharmaceutical technology.

In a preferred embodiment of the present invention the demethylated product of the aromatic methyl ether (III), *O*-desmethylvenlafaxine, is subjected to further reaction steps to obtain a pharmaceutical active compound.

The following examples illustrate the present invention and are not intended to limit the scope of the invention:

### Example 1: Synthesis of O-desmethylvenlafaxine

Venlafaxine hydrochloride (6.28 g, 20 mmol), K₂CO₃ (19.37 g, 140 mmol) and dimethylacetamide (40 mL) were charged in a 75 mL autoclave. 3-mercaptopropionic acid (8.48 g, 80 mmol) is slowly added to the reaction mixture while slowly stirring (CO₂ is releasing meanwhile). Reaction mixture is further gently stirred until CO₂ formation is completely stopped and the majority of crude substance is dissolved (about 10-30 min.). Autoclave is after that closed and dipped in a silicone oil bath, heated to 180 °C. Reaction mixture is stirred and heated at that temperature for six days. Autoclave is than removed from bath, cooled down to room temperature, opened and the content is poured into 200 mL of water. The mixture is stirred at least two hours until most solid (K₂CO₃) is dissolved. Insoluble residue (the light-grey mixture of unrelated venlafaxine and product) is filtered off and washed with water (2 x 100 mL). Crude material is transferred into 2 M NaOH (100 mL) where it is stirred for additional 2 hours to convert the product into sodium salt. The residue (unreacted venlafaxine) is filtered off and discarded, while filtrate is washed with MTBE (2 x 50 mL) and the organic phase discarded as well. Water phase is acidified with concentrated acetic acid to pH = 7.5. The precipitate is filtered off and washed with water (2 x 50 mL). The filtrate is again alkalified with 1 M NaOH to pH = 9.0. Some additional precipitate is formed which is also filtered off, washed with water (2 x 50 mL) and combined with the first precipitate. The combined crude product is vacuum dried at 50 °C (about 4 g of dry product) and crystallized from system ethyl acetate - ethanol 1 : 4 (100 mL). After filtering and vacuum drying at 50 °C, 3.28 g (62 %) of white crystalline product is obtained with HPLC chromatographic purity of 100.0 %.

### Example 2: Preparation of 4-hydroxy-1-naphthonitrile

A mixture of 4-methoxy-1-naphthonitrile (0.92 g, 5.0 mmol), potassium carbonate (4.15 g, 30 mmol), 3-mercaptopropionic acid (2.12 g, 20 mmol) and dimethylacetamide (25 mL) is stirred at 150 °C for 7 h and the solvent is removed under reduced pressure. Water is added (20 mL), followed by acetic acid (6.8 mL) and the product is extracted with MTBE (6x15 mL). The organic phases are combined, dried with sodium sulfate and concentrated under reduced pressure. The crude is purified by chromatography (silica gel; hexane : ethyl acetate = 92 : 8; 36 : 64) to yield 0.81 g (96 % yield) of 4-hydroxy-1-naphthonitrile. ¹H NMR (DMSO-*d*₆): δ 6.98 (d, 1 H; J = 8.0 Hz), 7.63 (m, 1 H), 7.76 (m, 1 H), 7.97 (d, 1 H; J = 8.2 Hz), 8.00 (d, 1 H; J = 8.2 Hz), 8.27 (d, 1 H; J = 8.4 Hz), 11.55 (s, 1 H).

### Example 3: Preparation of 4-hydroxy-3,5-dimethoxybenzonitrile.

A mixture of 3,4,5-trimethoxybenzonitrile (0.97 g, 5.0 mmol), potassium carbonate (4.15 g, 30 mmol), 3-mercaptopropionic acid (2.12 g, 20 mmol) and dimethylacetamide (25 mL) is stirred at 150 °C for 4.5 h and the solvent is removed under reduced pressure. Water is added (20 mL), followed by acetic acid (5 mL) and the product is extracted with MTBE (3 × 25 mL). The organic phases are combined, dried with sodium sulfate and concentrated under reduced pressure. The crude is purified by chromatography (silica gel; hexane : ethyl acetate = 50 : 50) to yield 0.40 g (45 % yield) of 4-hydroxy-3,5-dimethoxybenzonitrile. ¹H NMR (DMSO-*d*₆): δ 3.80 (s, 6H), 7.11 (s, 2H), 9.58 (s, 1 H).

### Example 4: Preparation of (4-hydroxyphenyl)(4-methoxyphenyl)methanone.

A mixture of 4,4'-dimethoxybenzophenone (1.21 g, 5.0 mmol), potassium carbonate (4.15 g, 30 mmol), 3-mercaptopropionic acid (2.12 g, 20 mmol) and dimethylacetamide (25 mL) is stirred at 150 °C for 2 h and the solvent is removed under reduced pressure. Water is added (5 mL), followed by acetic acid (10 mL) and the product is extracted with ethyl acetate (3 × 15 mL). The organic phases are combined, dried with sodium sulfate and concentrated under reduced pressure. The crude is purified by chromatography (silica gel; hexane : ethyl acetate = 92 : 8; 0 : 100) to yield 0.67 g (59 % yield) of (4-hydroxyphenyl)(4-methoxyphenyl)methanone. ¹H NMR (DMSO-*d*₆): δ 3.85 (s, 3H), 6.88 (m, 2H), 7.07 (m, 2H), 7.62 (m, 2H), 7.68 (m, 2H), 10.34 (s, 1 H).

### Example 5: Preparation of 4,4'-dihydroxybenzophenone.

A mixture of 4,4'-dimethoxybenzophenone (1.21 g, 5.0 mmol), potassium carbonate (8.3 g, 60 mmol), 3-mercaptopropionic acid (4.24 g, 40 mmol) and dimethylacetamide (50 mL) is stirred at 150 °C for 18 h and the solvent is removed under reduced pressure. Water is added (30 mL), followed by 37 % hydrochloric acid (8 mL) and the product is extracted with MTBE (3 × 25 mL) and ethyl acetate (2 × 25 mL). The organic phases are combined, dried with sodium sulfate and concentrated under reduced pressure. The crude is purified by chromatography (silica gel; hexane : ethyl acetate = 50 : 50), crystallized from water and dried under reduced pressure to yield 0.95 g (89 % yield) of 4,4'-dihydroxybenzophenone. ¹H NMR (DMSO-*d*₆): δ 6.87 (m, 4H), 7.60 (m, 4H), 10.29 (s, 2H).

Demethylation of various aromatic ethers with 3-mercaptopropionic acid is presented in Table 1.

**Table 1. Demethylation of various aromatic methyl ethers:**

| **Starting methyl ether** | **MPA (eq.)** | **K₂CO₃ (eq.)** | **T [°C]** | **Time [h]** | **Product** | **Yield [%]*** | **Conver. [HPLCA%]** |
|---|---|---|---|---|---|---|---|
| 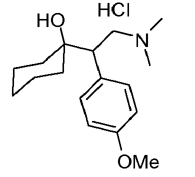 | 4 | 7 | 180 | 144 | 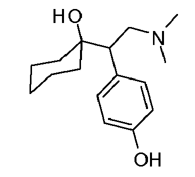 | 62 | 94 |
| 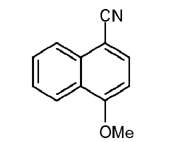 | 4 | 6 | 150 | 7 | 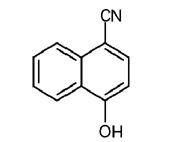 | 96 | >99 |
| 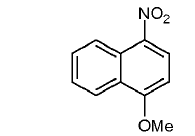 | 4 | 6 | 150 | 1 | 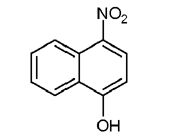 | 61^{§} | >99 |
| 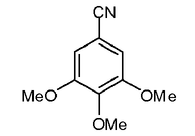 | 4 | 6 | 150 | 2 | 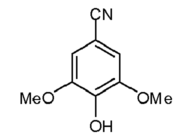 | 45 | >99 |
| 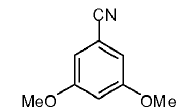 | 8 | 12 | 150 | 24 | 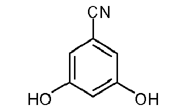 | 40^{§} | >99 |
| 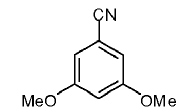 | 4 | 6 | 150 | 2 | 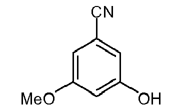 | 54^{§} | 78 |
| 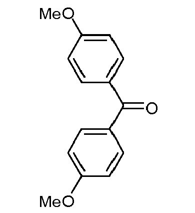 | 4 | 6 | 150 | 2 | 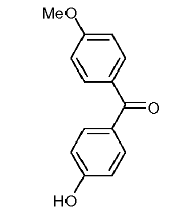 | 59 | 66 |
| 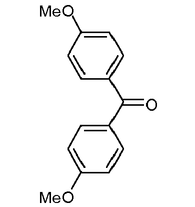 | 8 | 12 | 150 | 18 | 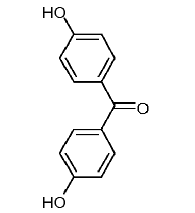 | 89 | 99 |

| | | | | | | | |
|---|---|---|---|---|---|---|---|
| * based on isolated product. ^{§}reaction and chromatographic isolation performed as in the examples 2-4. MPA = 3-mercaptopropionic acid | | | | | | | |

## Claims

1. A process for demethylating aromatic methyl ethers of the formula (I), 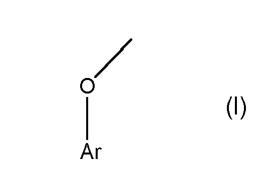 wherein Ar is selected from the group consisting of substituted or unsubstituted aryl and substituted or unsubstituted heteroaryl groups;
the process comprising reacting an aromatic methyl ether of the formula (I)
with 3-mercaptopropionic acid [formula (II)] or salts thereof

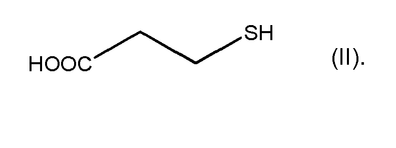

2. The process according to claim 1, wherein the reaction is carried out in the presence of a base in an organic solvent or mixtures comprising organic solvents.

3. The process according to claim 1 and 2, further comprising isolating the demethylated product from the reaction mixture.

4. The process according to claim 3, wherein the isolation step comprises a method of extraction, precipitation and/or crystallization, preferably wherein the demethylated product is separated from unreacted aromatic methyl ether of the formula (I) by precipitation in a basic mixture.

5. The process according to any one of the preceding claims, wherein the aromatic methyl ether (I) has n (n>1) methoxy groups as substituents on the aryl or heteroaryl groups, preferably 2, but only one methoxy group is selectively demethylated or wherein the aromatic methyl ether (I) has n (n>1) methoxy groups as substituents on the aryl or heteroaryl groups, preferably 2, and 2 and optionally up to n methoxy groups are demethylated.

6. The process according to any one of the preceding claims, wherein the Ar group consists of optionally substituted aryl groups comprising phenyl, naphthyl and anthryl or salts thereof, wherein the optional substituent is selected from the group of electron donating substituents consisting of respectively substituted or unsubstituted alkyls, amines, amides and/or is selected from the group of electron withdrawing substituents consisting of nitroxides, nitriles, carbonyls and carboxyls.

7. The process according to any one of the preceding claims, wherein Ar is substituted by a *p-*(dimethylamino)(1-hydroxycyclohexyl)methyl group.

8. The process according to claim 1, wherein Ar is substituted by a residue containing an amine group and the amine group is available in salt form, preferably as hydrochloride salt.

9. The process according to any of the preceding claims, wherein the reaction is carried out in a solvent selected from the group consisting of dimethylformamide, *N-*methyl-2-pyrrolidone, ethers, polyethers, polyethylene glycols, hydrocarbons, and *N,N'-*dimethylacetamide, preferably *N,N'*-dimethylacetamide.

10. The process according to any of the preceding claims, wherein a base used for the demethylation is selected from the group consisting of inorganic bases including carbonates, hydrides and hydroxides, and organic bases including pyridine, triethylamine.

11. The process according to any of the preceding claims, wherein the demethylation is carried out in a pressurizable reaction vessel, preferably an autoclave.

12. The use of 3-mercaptopropionic acid [formula (II)] or salts thereof for demethylation of venlafaxine or a salt thereof.

13. The process according to any of the preceding claims further comprising subjecting the demethylated product of the aromatic methyl ether (I) to further reaction steps to obtain a pharmaceutical active compound.

14. A process for the preparation of a pharmaceutical composition containing the demethylated product of the aromatic methyl ether (I) or a salt thereof as an active ingredient comprising:
(i) converting an aromatic methyl ether (I) or a salt thereof to the demethylated product or salt thereof by a process according to any one of claim 1 to 11;
(ii) optionally further reaction according to claim 13;
(iii) optionally forming a finally suitable salt;
(iv) formulating the obtained demethylated product or salt thereof with at least one pharmaceutical acceptable excipient.

15. A process for the preparation of a pharmaceutical composition containing *O*-desmethylvenlafaxine or a salt thereof as an active ingredient comprising:
(i) converting venlaflaxine or a salt thereof to *O*-desmethylvenlaflaxine or salt thereof by a process according to any one of claim 1 to 11 or the use according to claim 12;
(ii) optionally forming a finally suitable salt;
(iii) formulating the obtained *O*-desmethylvenlaflaxine or salt thereof with at least one pharmaceutical acceptable excipient.
